# EUROPÄISCHE PATENTSCHRIFT

(11) **EP 3 586 918 B1**
(45) Veröffentlichungstag und Bekanntmachung des Hinweises auf die Patenterteilung: **05.04.2023**
(21) Anmeldenummer: 19181989.5
(22) Anmeldetag: 24.06.2019
(51) Int. Cl.: A61N 1/372, A61N 5/06, A61K 48/00, A61N 1/362, A61N 1/05, A61B 5/00, A61N 1/368, A61N 5/073

(54) **VORRICHTUNG ZUR AKTIVIERUNG VON ZELLSTRUKTUREN MITTELS ELEKTROMAGNETISCHER ENERGIE**
DEVICE FOR ACTIVATING OF CELL STRUCTURES BY MEANS OF ELECTROMAGNETIC ENERGY
DISPOSITIF D'ACTIVATION DES STRUCTURES CELLULAIRES AU MOYEN DE L'ÉNERGIE ÉLECTROMAGNÉTIQUE

(30) Priorität: 25.06.2018 DE 102018115180; 24.10.2018 DE 102018126468
(43) Veröffentlichungstag der Anmeldung: 01.01.2020
(73) Patentinhaber: BIOTRONIK SE & Co. KG, 12359 Berlin (DE)
(72) Erfinder: Dörr, Thomas, 12437 Berlin (DE); Weiss, Ingo, 12435 Berlin (DE)
(74) Vertreter: Biotronik Corporate Services SE

(56) Entgegenhaltungen:
- WO-A1-2016/019075
- WO-A1-2016/023126
- WO-A1-2017/109103
- WO-A2-2012/052727
- US-A1- 2010 190 229
- US-A1- 2010 202 966

## Beschreibung

Die Erfindung beschreibt eine Vorrichtung und Methode zur Aktivierung von Zellstrukturen mittels elektromagnetischer Energie.

Seit langem bekannt sind Stimulatoren zur Anregung von menschlichem oder tierischem Gewebe durch elektrischen Strom. Elektrische Stimulation von Nervengewebe bewirkt die Aktivierung von Aktionspotentialen, die in der medizinischen Therapie und Diagnostik genutzt wird. Beispiele für implantierbare Stimulatoren sind u.a. implantierbare Herzschrittmacher und -Defibrillatoren, Rückenmarkstimulatoren, Vagusnervstimulatoren, Hirnschrittmacher.

Elektrische Stimulationsvorrichtungen sind galvanisch an menschliches oder tierisches Gewebe gekoppelt und messen elektrische Potentiale und/oder stimulieren das Gewebe durch elektrische Stromabgabe zur Auslösung von Aktionspotentialen. Nachteile der elektrischen Stimulation ergeben sich unter anderem durch die notwendige galvanische Verbindung zum Gewebe. Die direkte Kontaktierung Stimulationselektroden mit dem Gewebe und Einkopplung von Strom können z.B. Nekrosen, elektrische Nachpotentiale, die die Sensingfunktionen limitieren, Crosstalk (d.h. Interferenzen bei paralleler Stimulation über verschiedene Stimulationspfade), ungewollte externe Induktion von Therapieströmen (z.B. bewirkt durch die hochfrequenten Magnetfelder innerhalb eines Magnetresonanztomografen), eventuelle Schmerzereignisse, Elektroporation oder ungewollte Stimulation in der Nähe befindlichen Gewebes (z.B. kann bei Herzstimulatoren der Nervus Phrenicus ungewollt mitstimuliert werden) verursachen. Weitere Nachteile ergeben sich durch die mit dem Therapiestrom verbundenen Einrichtungen zur Stromgenerierung, die aufwändig und voluminös sein können (z.B. Ladeschaltung bei einem implantierbaren Kardioverter-Defibrillator) und Limitationen in der Erreichbarkeit des Zielgewebes aufweisen.

Seit einigen Jahren ist die genetische Manipulation von Gewebe zur Generierung einer Erregbarkeit mittels elektromagnetischer Wellen Gegenstand der wissenschaftlichen Diskussion und Forschung. Dabei ist es gelungen, Zielgewebe genetisch so zu verändern, dass mittels elektromagnetischen Wellen Aktionspotentiale evoziert werden können.

Eine dieser Methoden wird als optogenetische Manipulation bezeichnet. Diese wird beispielsweise beschrieben in
- Lung MS, Pilowsky P, Goldys EM., "Activation of the Mammalian cells by using light-sensitive ion channels." Methods Mol Biol. 2012;875:241-51; die Publikation beschreibt optogenetische Gewebemanipulation zum Zwecke der Auslösung von Aktionspotentialen mittels Licht im sichtbaren Spektrum;
- Wang et al. "Optogenetic Control of Heart Rhythm by Selective Stimulation of Cardiomyocytes Derived from Pnmt+ Cells in Murine Heart", Januar 2017 in Scientific Reports.

US 2010/0202966 A1 beschreibt eine Vorrichtung zum Erfassen einer Konzentration eines Analyten in einem Subjekt. Die Vorrichtung umfasst ein Gehäuse, das zur Implantation in das Subjekt geeignet ist. Das Gehäuse umfasst einen optischen Detektor, der zur Erfassung eines Levels eines Fluoreszenzresonanzenergietransfers (Fluorescence Resonance Energy Transfer, FRET) ausgelegt ist.

WO 2012/052727 A2 beschreibt eine in vitro-Population von Zellen, umfassend (i) ein Motoneuron, umfassend ein Transgen; wobei das Transgen ein lichtempfindliches Protein codiert, das in der Lage ist, ein Aktionspotential bei Beleuchtung mit Licht einer verwandten Wellenlänge auszulösen oder zu unterdrücken; und (ii) einen Astrozyten.

US 2010/0190229 A1 beschreibt Methoden und Vorrichtungen zur Stimulation von Zellen in vivo unter Verwendung von Licht. In einem Beispiel wird ein Verfahren zur Stimulation von Zellen mit Licht unter Verwendung eines lichtempfindlichen Proteins beschrieben. WO 2017/109103 A1 beschreibt einen Temperatursensor, umfassend einen Lichtemitter, eine elektrische Schaltung zum Anlegen einer Sperrvorspannung an den Lichtemitter und zum Messen eines Sperrstroms, und Mittel zum Berechnen einer Temperatur aus dem gemessenen Sperrstrom.

US 2009/0088680 A1 beschreibt ein katheterbasiertes System zur Einbringung eines Lichtleiters in das Herz, wobei das Herzgewebe zuvor mittels optogenetischer Manipulation behandelt wurde. Um den Lichtleiter in das Herzinnere zu befördern, wird ein Gefäßzugang beim Patienten gelegt, ein Katheter wird in das Herz vorgeschoben und der Lichtleiter durch den Katheter in das Herzinnere gebracht. Über den Lichtleiter wird Licht in das Herz geleitet und eine endokardiale Stimulation des manipulierten Gewebes durchgeführt.

Ein Nachteil der in US 2009/0088680 A1 beschriebenen Methode besteht darin, dass sie durch einen Arzt ambulant durchgeführt werden und der Lichtleiter muss von einem externen Gerät betrieben werden muss. Nachteile entstehen auch durch den venösen Gefäßzugang und die Katheterisierung wie zum Beispiel Infektionen und Gefäßverschlüsse.

Die Aufgabe der Erfindung ist es, in einer dauerhaft implantierbaren Vorrichtung Aktionspotentiale an vorbehandelten tierischen und/oder menschlichen erregbaren Zellstrukturen detektieren, ohne dass es einer galvanischen Einprägung elektrischem Stromes in besagte Zellstrukturen bedarf.

Die Aufgabe der Erfindung ist es, in einer dauerhaft implantierbaren Vorrichtung Aktionspotentiale an vorbehandelten tierischen und/oder menschlichen erregbaren Zellstrukturen zu evozieren, modulieren, terminieren und/oder zu inhibieren ohne dass es einer galvanischen Einprägung elektrischem Stromes bedarf.

Die Erfindung löst die Aufgabe, zelluläre Aktionspotentiale zu detektieren, ohne dass eine galvanische Verbindung zum Zielgewebe vorhanden sein muss.

Eine Aufgabe der vorliegenden Erfindung ist die Bereitstellung einer Vorrichtung und eines Systems, das die genannten Nachteile nicht aufweist.

Eine weitere Aufgabe der vorliegenden Erfindung besteht in der Bereitstellung einer Vorrichtung und eines Systems für die Stimulation von Gewebe mittels elektromagnetischen Wellen zur Auslösung von Aktionspotentialen, wobei die Vorrichtung eine dauerhafte Stimulation ermöglicht, ohne dass ein permanenter Gefäßzugang, externe Geräte oder eine ambulante Sitzung beim Arzt erforderlich ist.

Die Aufgabe wird erfindungsgemäß durch die Merkmale des unabhängigen Anspruchs gelöst. Günstige Ausgestaltungen und Vorteile der Erfindung ergeben sich aus den weiteren Ansprüchen und der Beschreibung.

Die Erfindung ist durch Anspruch 1 definiert. Bevorzugte Ausführungsbeispiele werden durch die abhängigen Ansprüche definiert.
- Fig. 1: Schematische Darstellung einer Stimulationsvorrichtung im implantierten Zustand mit einem externen Gerät
- Fig. 2: Schematische Darstellung einer Methode zur Behandlung einer Bradykardie mittels optischer Stimulation
- Fig. 3: Schematische Darstellung einer Methode zur Behandlung einer tachykarden Herzrhythmusstörung in Form einer ventrikulären oder atrialen Tachykardie
- Fig. 4: Schematische Darstellung einer Methode zur Behandlung eines Vorhofflimmerns
- Fig. 5: Schematische Darstellung einer Methode zur Behandlung eines Kammerflimmerns
- Fig. 6: Schematische Darstellung einer Methode zur Anwendung der kardialen Resynchronisationstherapie mittels elektromagnetischer Wellen
- Fig. 7: Schematische Darstellung einer Neurostimulationsmethode mittels elektromagnetischer Wellen
- Fig. 8: Schematische Darstellung einer Ausführungsform eines implantierbaren Stimulators
- Fig. 9: Implantierbarer Stimulator gemäß Figur 8 mit einer alternativen Fixiervorrichtung
- Fig. 10: Blockschaltbild des implantierbaren Stimulators

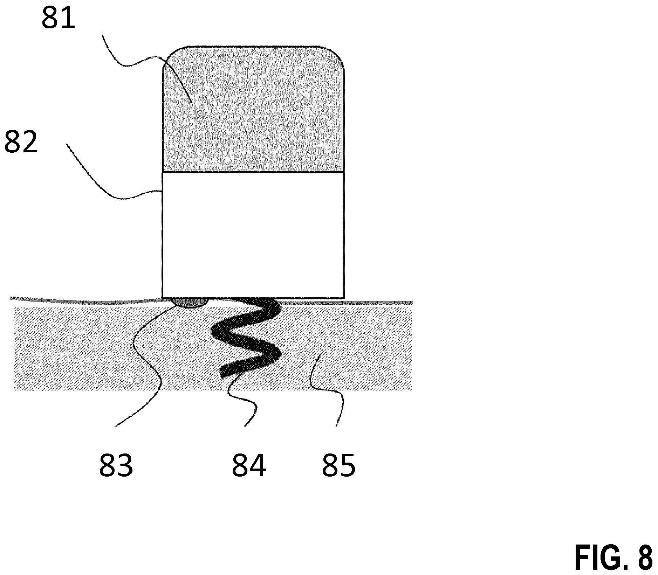

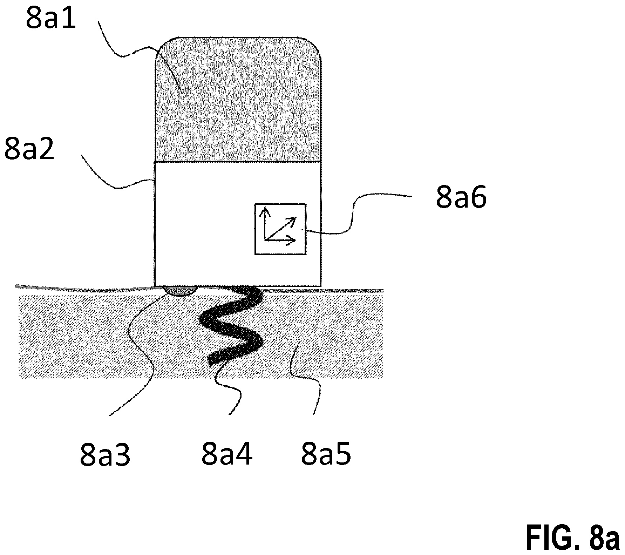

- Fig. 8a: Schematische Darstellung einer Stimulationsvorrichtung mit Therapieerfolgskontrolle

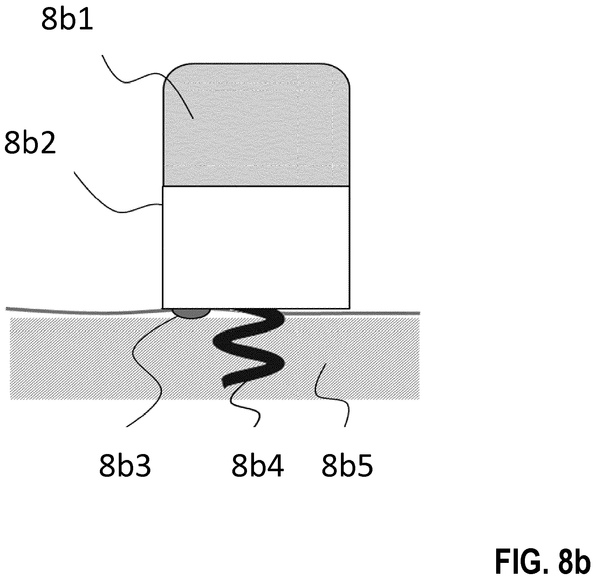

- Fig. 8b: Schematische Darstellung einer Stimulationsvorrichtung mit Defibrillationsfunktion

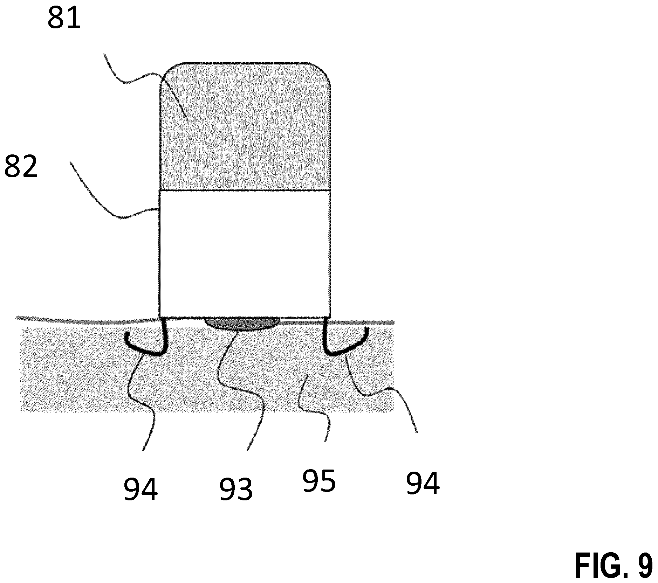

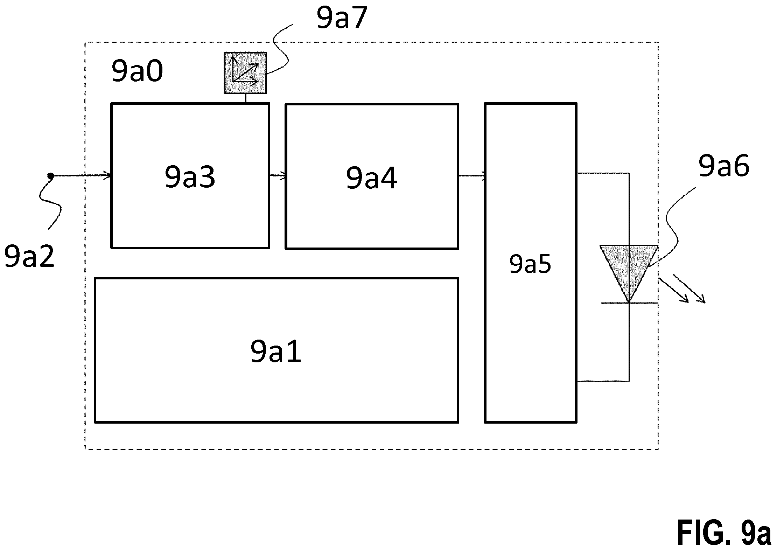

- Fig. 9a: Blockschaltbild einer Stimulationsvorrichtung

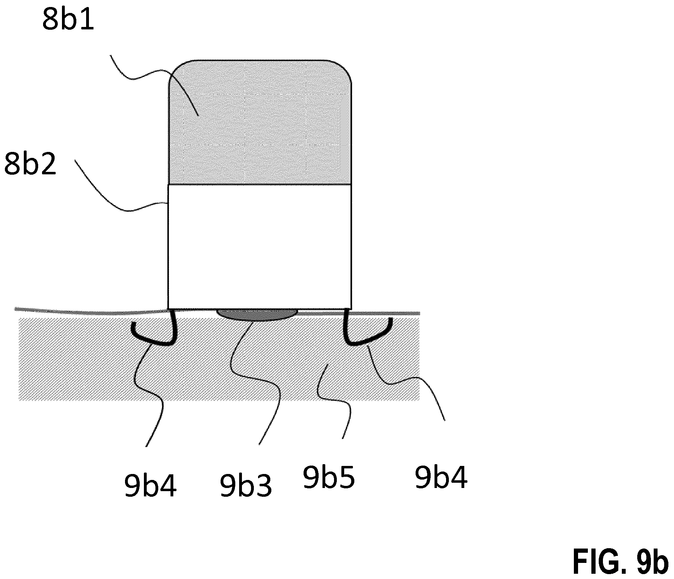

- Fig. 9b: Implantierbarer Stimulator gemäß Figur 8b mit einer alternativen Fixiervorrichtung

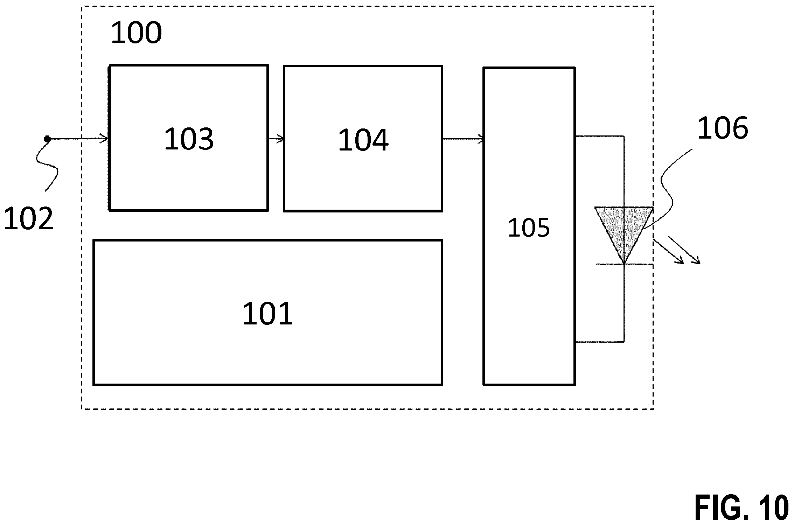

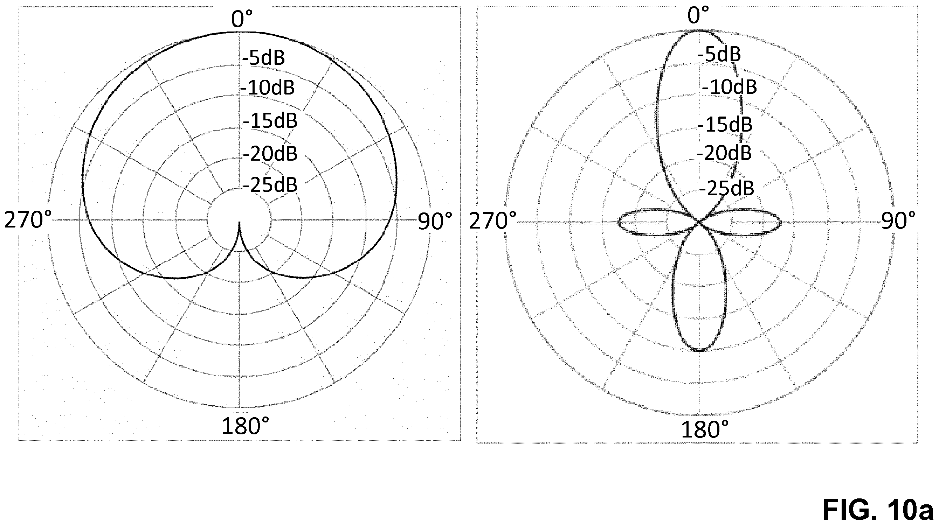

- Fig. 10a: zeigt beispielhaft zweidimensionale Charakteristiken elektromagnetischer Strahlers mit räumlich selektiver Wirkung.

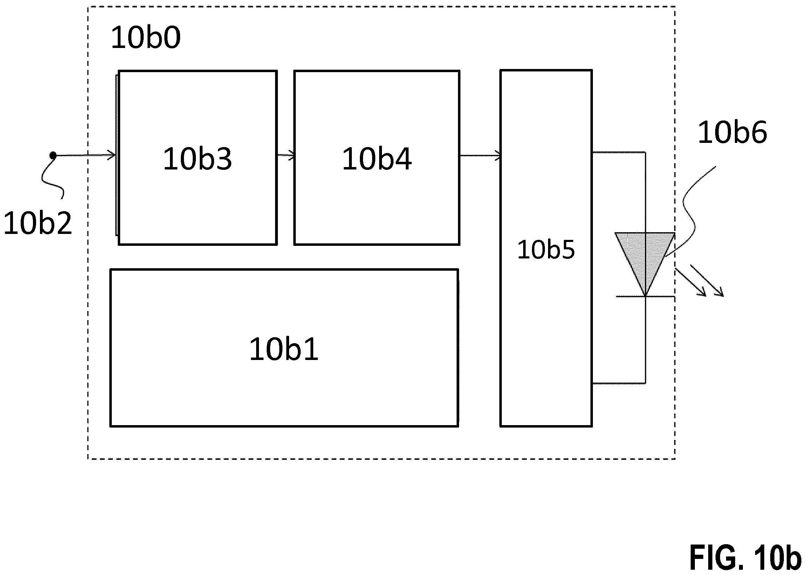

- Fig. 10b: Blockschaltbild der Vorrichtung nach Figur 8b

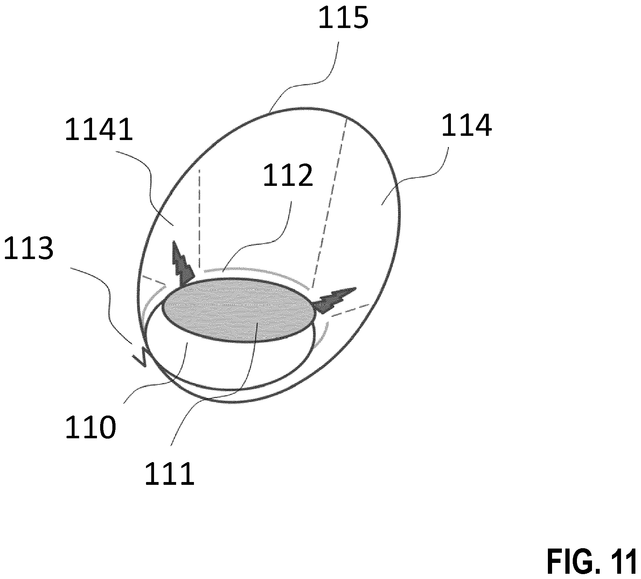

- Fig. 11: bis 14 zeigen Realisierungen des Stimulationssystems mit Selektionsmittel, das dazu ausgelegt ist, den Bereich des besagten Gewebes für die Stimulation zu wählen

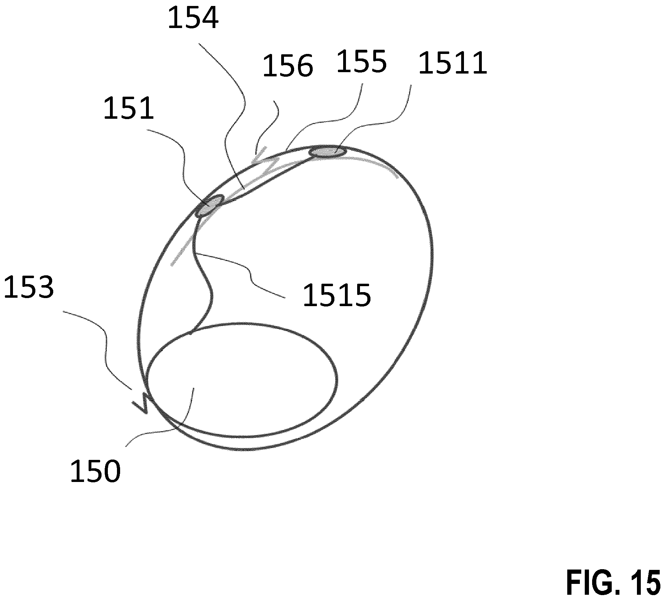

- Fig. 15: Realisierung des Stimulationssystems mit Selektionsmittel, das dazu ausgelegt ist, den Bereich des besagten Gewebes für die Stimulation zu wählen, wobei das Stimulationssystem über Träger verfügt

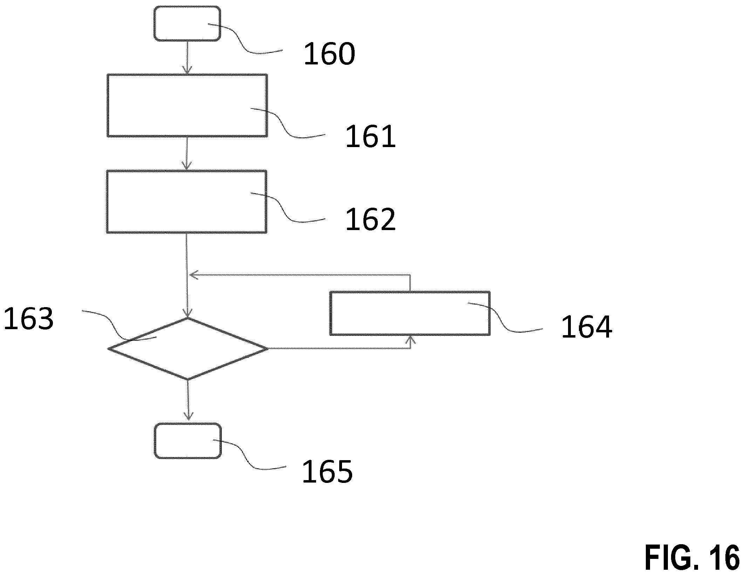

- Fig. 16: Ablaufplan der Methode für das Stimulationssystem

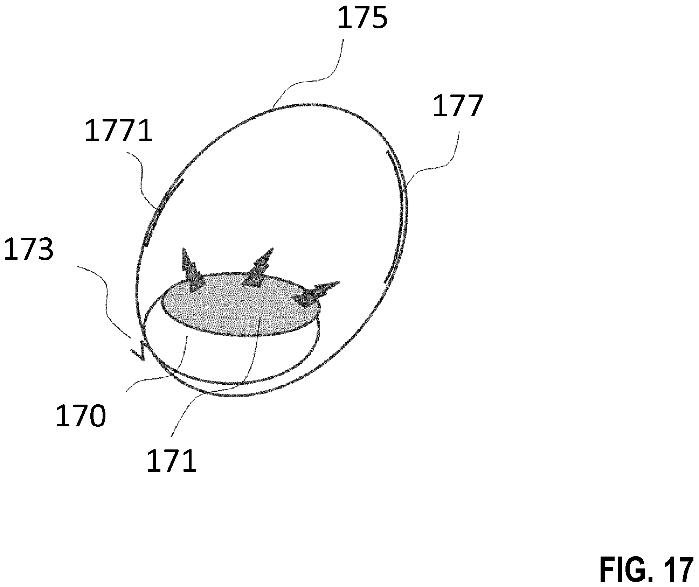

- Fig. 17: Schematische Darstellung der lokalen Vorbehandlung zur Erzeugung empfindsamer Bereiche für elektromagnetische Strahlung

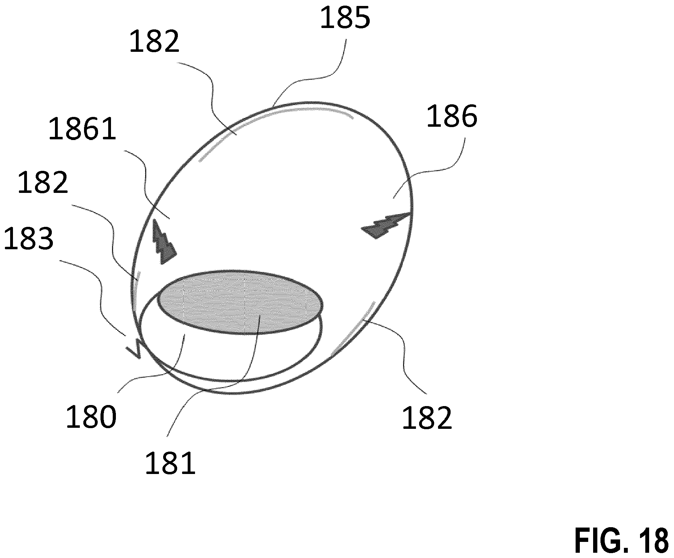

- Fig. 18: Schematische Darstellung der lokalen Vorbehandlung zur Erzeugung einer Maskierung ggf. empfindsamer Bereiche für elektromagnetische Strahlung.

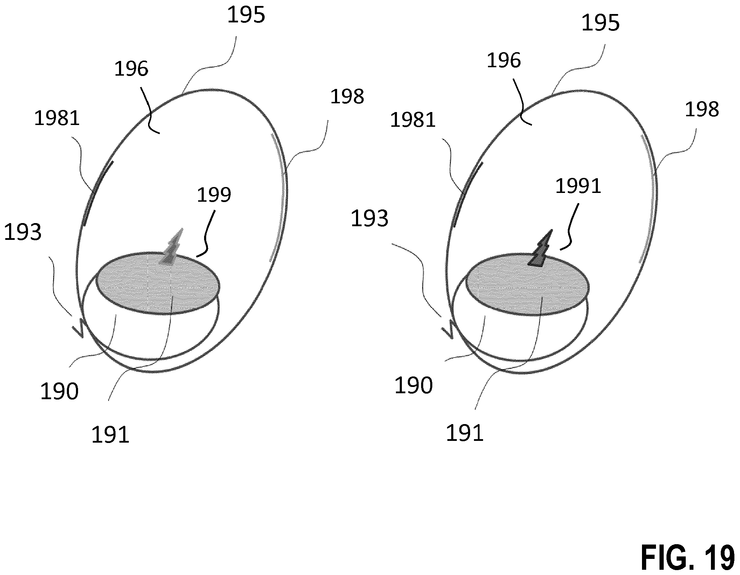

- Fig. 19: Beispiel mit lokal vorbehandelten Bereichen für die Stimulation mittels elektromagnetischer Strahlung

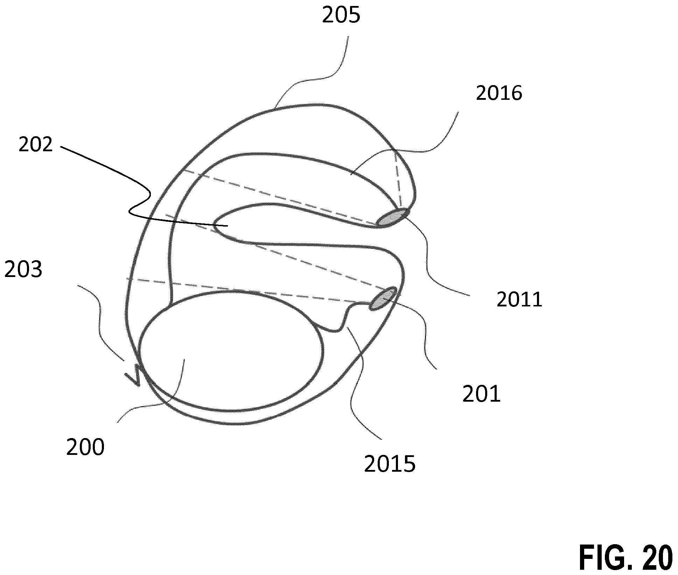

- Fig. 20: Schematische Darstellung der selektiven Therapie durch Platzierung der Aktuatoren, so dass anatomische Beschaffenheiten des Gewebes zur Abschattung genutzt werden

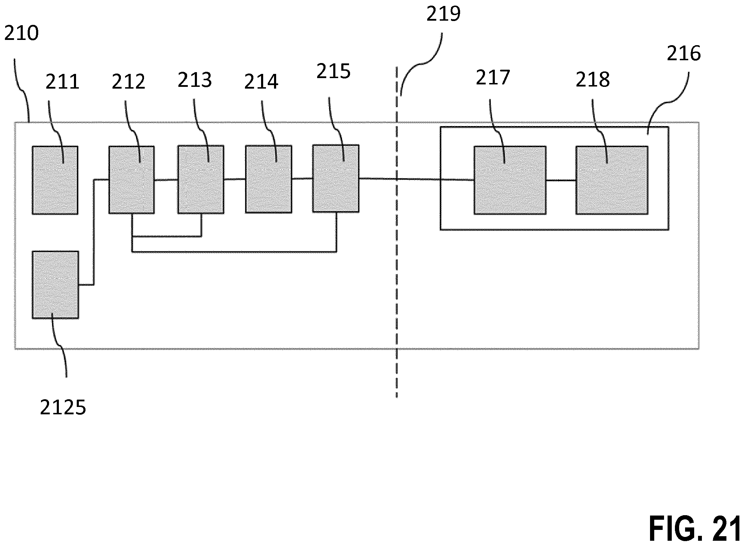

- Fig. 21: Blockschaltbild eines Stimulators

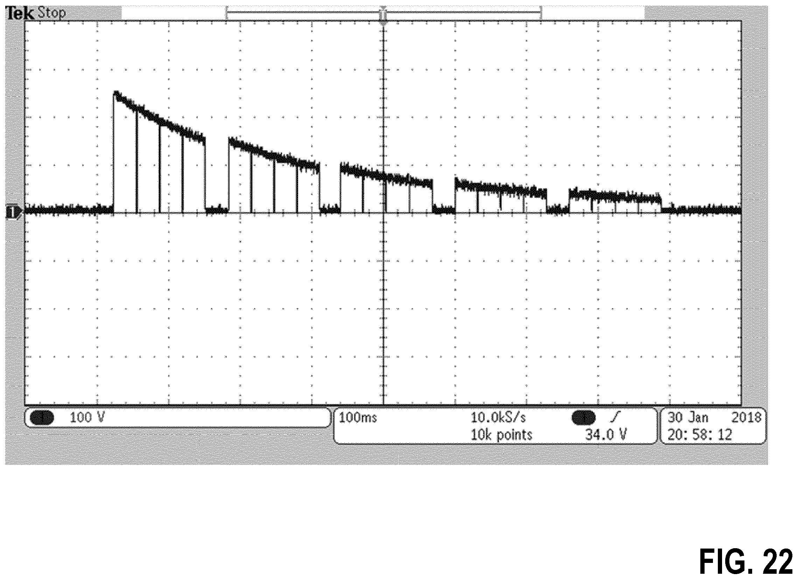

- Fig. 22: Schematische Darstellung der Pulsentladung eines ICDs, die einen Stimulator mit Therapiemöglichkeit mittels elektromagnetischer Strahlung zur Abgabe einer gepulsten Therapie ansteuert.

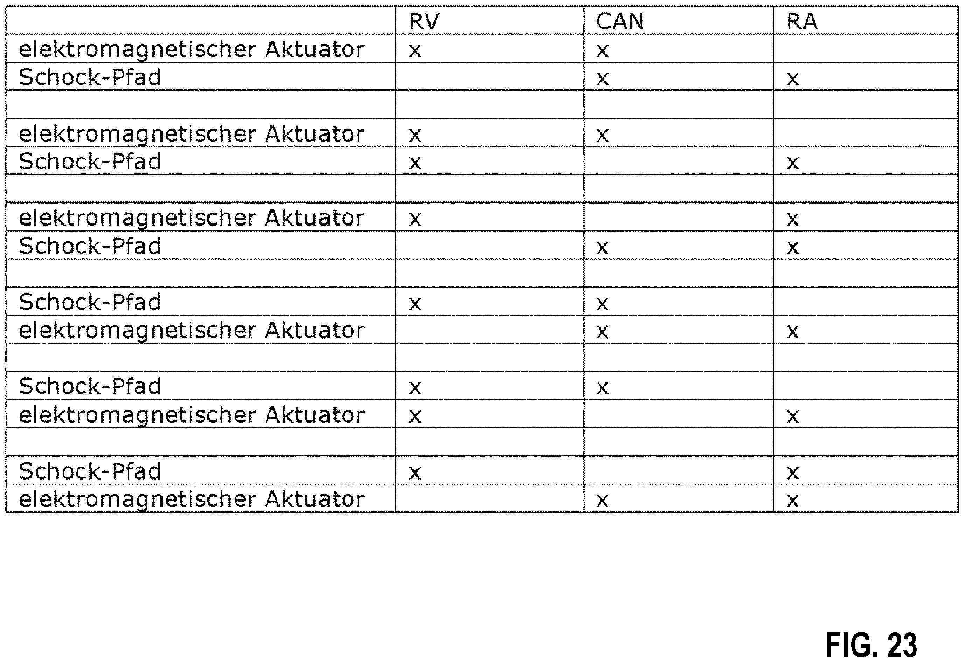

- Fig. 23: Tabelle, aufzeigend beispielhafte Kombinationen von Stimulations- und Schockvektoren zwischen rechtem Ventrikel, Gehäuse der Stimulationsvorrichtung und rechtem Atrium.

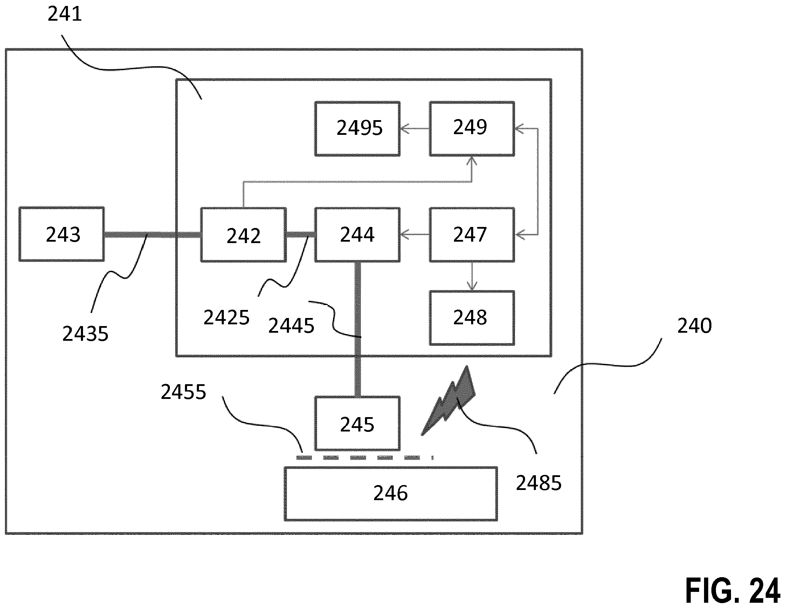

- Fig. 24: Schematische Darstellung einer Vorrichtung umfassend ein Applikationsmittel zur Abgabe einer Substanz an Gewebe.

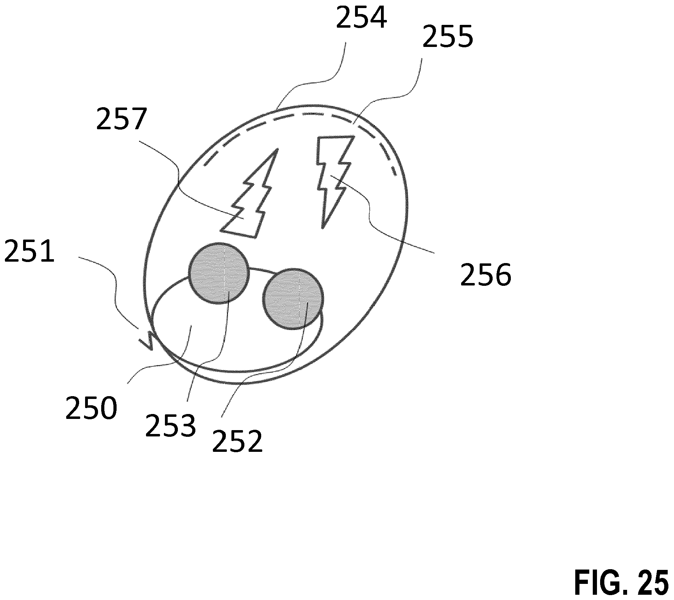

- Fig. 25: Schematische Darstellung der erfindungsgemäßen implantierbaren Vorrichtung nach einem Ausführungsbeispiel, wobei die Vorrichtung mit einer Fixiereinrichtung am Gewebe fixiert ist

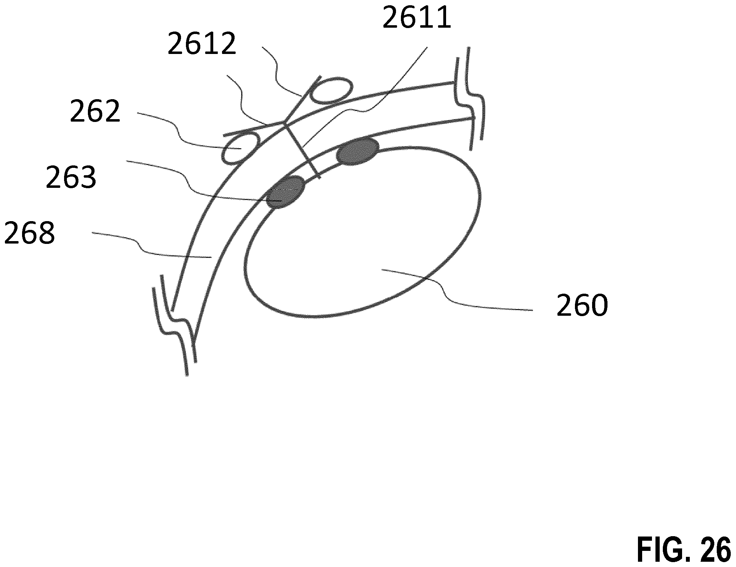

- Fig. 26: Schematische Darstellung der erfindungsgemäßen Vorrichtung gemäß einem Realisierungsbeispiel. Die Vorrichtung verfügt über Aktuatoren bzw Sensoren für elektromagnetische Strahlung, die in der Fixiereinheit eingebaut sind.

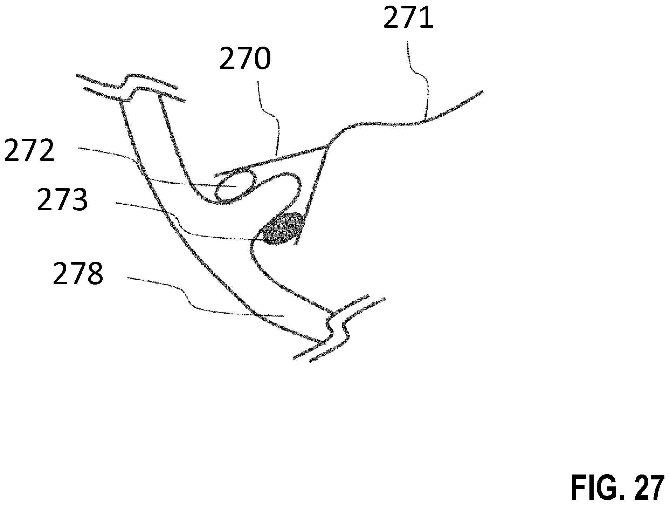

- Fig. 27: Schematische Darstellung der erfindungsgemäßen Vorrichtung gemäß einem weiteren Realisierungsbeispiel. Die Vorrichtung verfügt über Aktuatoren bzw Sensoren für elektromagnetische Strahlung, die in der Fixiereinheit eingebaut sind.

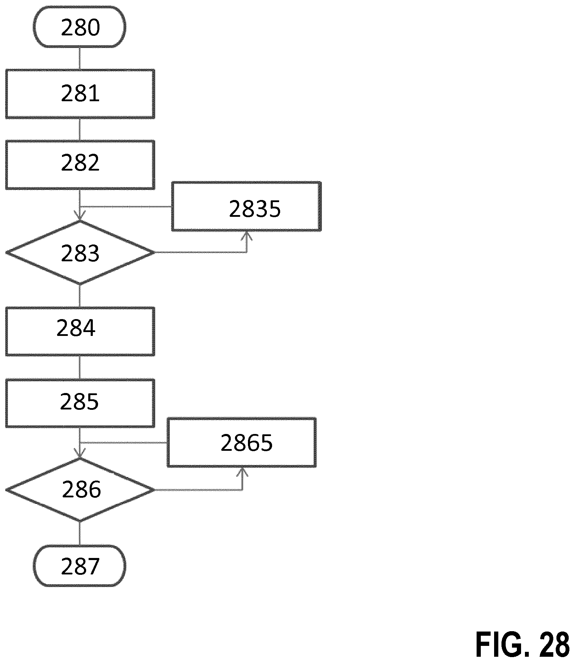

- Fig. 28: zeigt beispielhaft einen bevorzugten Signalverarbeitungsablauf

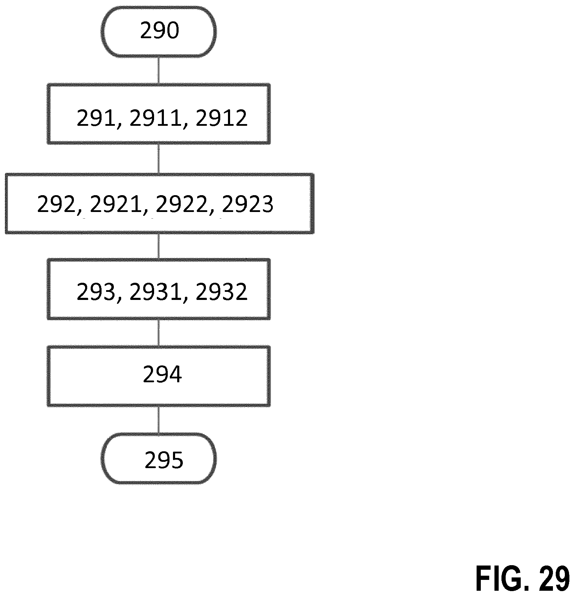

- Fig. 29: zeigt beispielhaft einen Signalverarbeitungsablauf

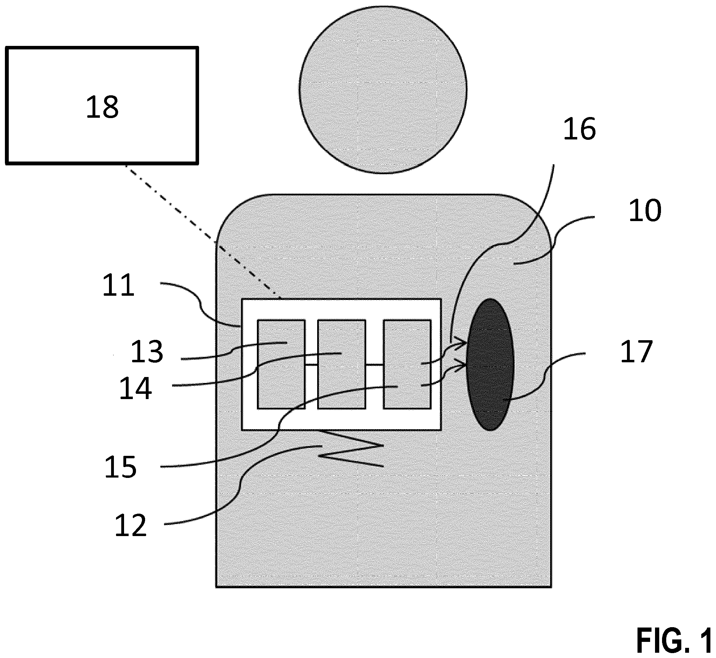

In Figur 1 ist ein implantierbarer Stimulator dargestellt. Dieser wird in das Körpergewebe des Patienten 10 implantiert und umfasst aus einem hermetisch versiegelten Gehäuse 11. Diese ist hier mit einer Fixiervorrichtung 12 im Körpergewebe verankert. Das Implantatgehäuse 11 beinhaltet eine Energiequelle 13, eine Steuereinheit 14 und einen Aktuator 15, der ausgebildet ist, eine elektromagnetische Strahlung 16 z.B. im Bereich des sichtbaren Lichtes an ein zuvor beispielsweise mittels genetischer, speziell optogenetischer Manipulation vorbehandeltes Körpergewebe abgeben zu können und beispielsweise ein Aktionspotential zu evozieren, d.h. eine Stimulation mittels elektromagnetsicher Strahlung auszulösen Zusätzlich kann das Implantat mittels eines externen Geräts 18, z.B. einer Telemetrieeinheit ausgelesen und/oder programmiert werden.

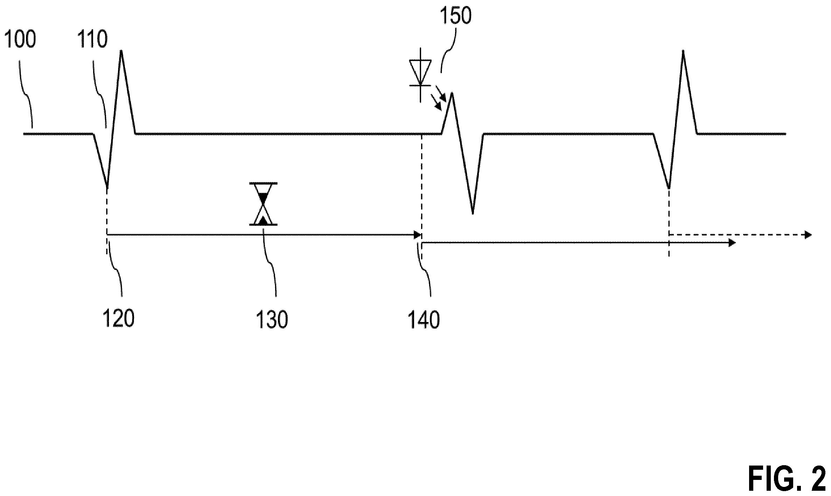

In der Figur 2 ist eine Methode zur Behandlung einer Bradykardie mittels Stimulation von genetisch manipuliertem Gewebe, bevorzugt optogenetisch manipuliertem Gewebe, illustriert. Im schematisierten EKG 100 ist zunächst eine reguläre Herzaktion 110 in Form eines intrinsischen QRS-Komplex dargestellt. Dieser wir bei dem vorgeschlagenen Verfahren zunächst registriert 120 und anschließend ein Zeitintervall 130 gestartet, dass einer erwarteten Herzrate entspricht. Läuft dieses Erwartungsintervall ab 140, ohne dass erneut ein intrinsischer QRS-Komplex registriert wird, so wird eine optische Stimulation 150 ausgelöst, die im vorzugsweise optogenetisch vorbehandelten Myokardgewebe einen stimulierten QRS-Komplex auslöst.

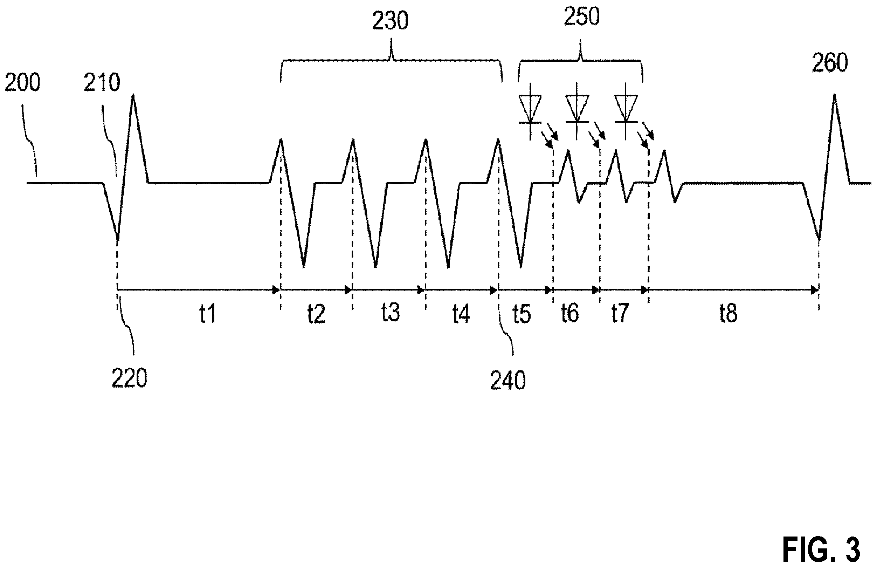

In der Figur 3 ist eine Methode zur Behandlung einer tachykarden Herzrhythmusstörung in Form einer ventrikulären oder atrialen Tachykardie illustriert. Im schematischen EKG 200 ist zunächst eine reguläre Kontraktion 210 einer Herzkammer dargestellt. Zur Überwachung des Herzrhythmus wir jede dieser Kontraktionen registriert und startet eine Zeitmessung bis zur nächsten registrierten Kontraktion (11... t8). Im dargestellten Beispiel setzt nun eine zu schnelle Herzrate in Form einer Tachykardie 230 ein, die zunächst über einige Zyklen registriert und bestätigt wird 240. Nach Bestätigung 240 wird eine vorbestimmte optische Stimulationssequenz 250 an das vorzugsweise vorbehandelte Myokard abgegeben, welche eine Reihe von stimulierten Erregungen verursacht, die in der Regel die Tachykardie beenden und einen regulären Rhythmus wieder herstellen 260.

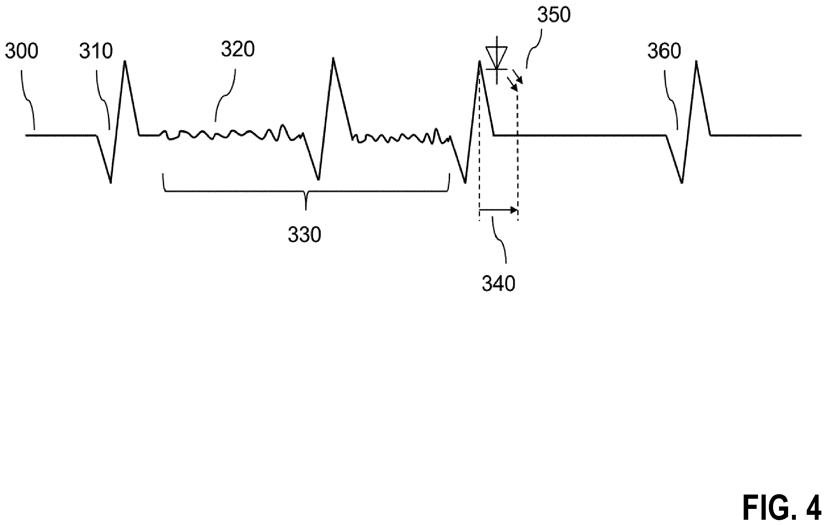

In der Figur 4 ist eine Methode zur Behandlung eines Vorhofflimmerns illustriert. Im schematischen EKG 300 ist zunächst eine QRS-Komplex in Sinusrhythmus 310 dargestellt. Anschließend setzt ein Vorhofflimmern 320 spontan ein. Dieses wird zunächst registriert und bestätigt 330. Mit der Bestätigung erfolgt die Abgabe einer optischen Stimulation 350 der Vorhöfe des vorbehandelten Herzens, jedoch synchronisiert und zeitversetzt zu einem QRS-Komplex (Kardioversion). Hiermit wird das Vorhofflimmern terminiert und ein regulärer Sinusrhythmus wird wieder hergestellt 360.

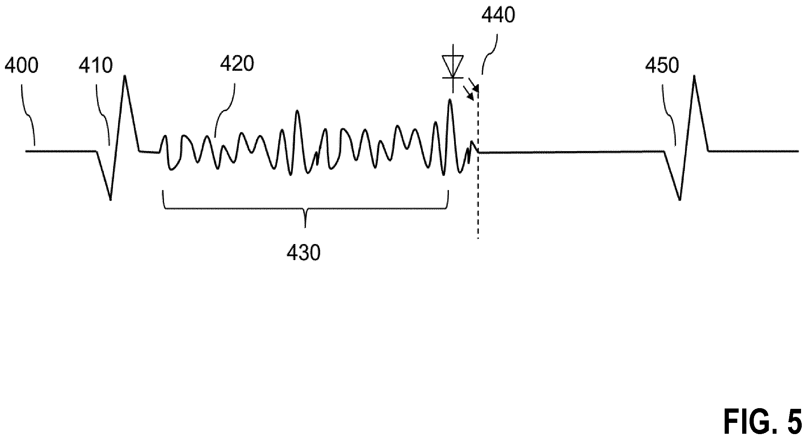

In der Figur 5 ist eine Methode zur Behandlung eines Kammerflimmerns illustriert. Im schematischen EKG 400 ist zunächst eine QRS-Komplex in Sinusrhythmus 410 dargestellt. Anschließend setzt spontan Kammerflimmern ein 420, welches zunächst registriert und anschließend bestätigt wird 430. Mit der Bestätigung wird ein großflächige optische Stimulation 440 im Bereich der vorzugsweise optogenetisch vorbehandelten Ventrikel ausgelöst, welche das Kammerflimmern terminiert und einen Sinusrhythmus 450 wiederherstellt.

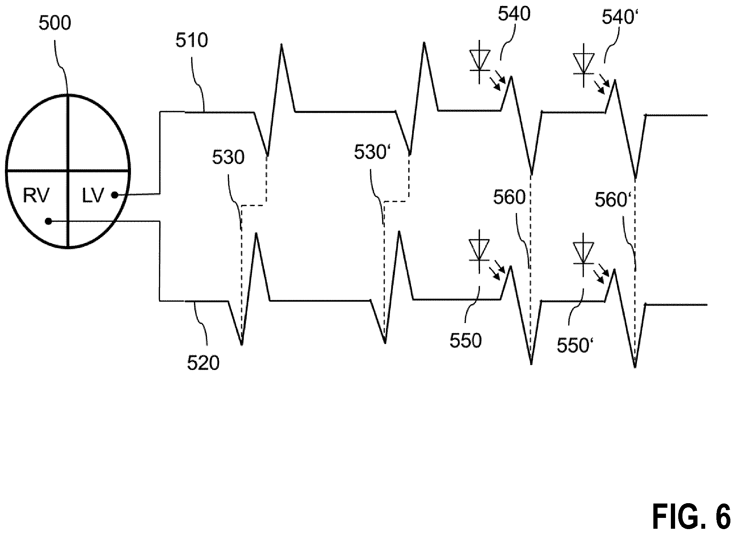

In der Figur 6 ist eine kardiale Resynchronisationsmethode illustriert zur Stimulation von genetisch manipuliertem Gewebe, bevorzugt optogenetisch manipuliertem Gewebe. Dargestellt sind schematisch die intrakardialen EKG-Ableitungen aus beiden Ventrikeln des Herzen 500: Linker Ventrikel (LV; 510) und rechter Ventrikel (RV, 520). Die EKG Ableitungen zeigen zunächst eine zeitliche Verzögerung 530 der linksventrikulären Herzaktionen gegenüber der rechtsventrikulären Herzaktionen. Dieser Versatz ist Ausdruck eines sogenannten Linksschenkelblocks, der bei fortgeschrittener struktureller Schädigung des Herzmuskels einer Therapie in Form einer Resynchronisation beider Ventrikel bedarf. Diese Resynchronisation erfolgt hier mittels simultaner oder quasisimultaner optischer Stimulation 540,550 beider Ventrikel des Herzens, so dass zeitgleich eine mechanische Kontraktion 560 beider Ventrikel dauerhaft hergestellt wird.

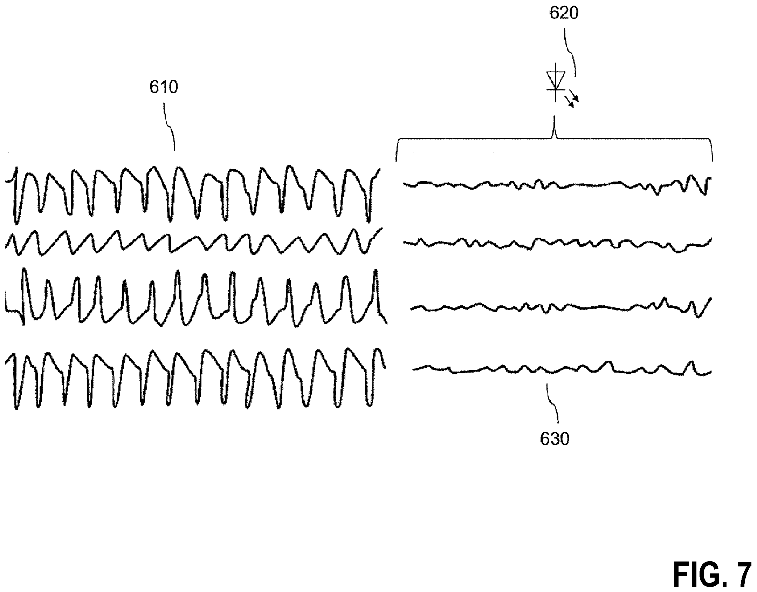

In der Figur 7 ist beispielhaft eine Neurostimulation von genetisch manipuliertem Gewebe, bevorzugt optogenetisch manipuliertem Gewebe, illustriert. Im gezeigten Beispiel ist auf der linken Seite ein EEG-Ausschnitt 610 eines epileptischen Anfalls abgebildet. Zu dessen Behandlung wird eine optische Stimulationssequenz 620 an eine oder mehrere vorzugsweise optogenetisch vorbehandelte Hirnregionen abgegeben, um eine Terminierung des epileptischen Anfalls herbeizuführen 630. Diese Darstellung soll die Methode der optischen Neurostimulation stellvertretend für alle weiteren Applikationen illustrieren, so z.B. Rückenmarksstimulation, weitere Applikationen der Deep Brain Stimulation oder kortikale Stimulation, isolierte Nervenstimulation, Muskelstimulation etc.

In der Figur 8 ist eine mögliche Ausführungsform eines implantierbaren Stimulators dargestellt. Dieser besteht aus einer Batterie 81 und einer Implantatselektronik 82, die die Komponenten des Blockschaltbildes aus Figur 10 beinhaltet, beides in einem hermetischen Gehäuse untergebracht. An der Unterseite des Gehäuses ist eine Lichtquelle 83 angebracht, die ebenfalls hermetisch versiegelt ist, jedoch so, dass das Frequenzspektrum dieser Lichtquelle die hermetische Versiegelung durchdringen und das Zielgewebe -hier Myokard- 85 erregen kann. Die Fixierung des Implantates erfolgt hier mit einer Helix 84 im Myokard 85.

In der Figur 9 ist der implantierbare Stimulator aus Figur 8 bestehend aus einer Batterie 81 und einer Implantatselektronik 82 dargestellt, jedoch mit einer alternativen Fixiervorrichtung 94, 94` in Form von seitlich angebrachten Widerhaken aus z.B. Nitinol. An der Unterseite des Gehäuses ist eine Lichtquelle 93 angebracht, die hermetisch versiegelt ist, derart, dass das Frequenzspektrum dieser Lichtquelle die hermetische Versiegelung durchdringen und das Zielgewebe -hier Myokard- 95 erregen kann.

In der Figur 10 ist das Blockschaltbild des implantierbaren Stimulators 100 dargestellt. Dieses Umfasst eine Energiequelle 101, ein Sensorinterface 102 zur Wahrnehmung eines eine Herzaktion repräsentierenden Merkmals, verbunden mit einer Wahrnehmungseinheit 103, die wiederum die Wahrnehmung von Herzaktionen an die angeschlossene Steuereinheit 104 signalisiert. Diese Steuereinheit 104 ist mit einem Therapiegenerator 105 verbunden, welcher bei Erhalt eines Triggersignals von der Steuereinheit 104 eine an den Therapiegenerator angeschlossene LED 106 anregt und damit ein das Myokardgewebe stimulierendes Lichtsignal aussendet. Der Therapiegenerator 105 kann dabei das Lichtsignal in seiner Intensität, Dauer, Signalform und Farbe variieren.

In der Figur 8a ist eine mögliche Ausführungsform eines implantierbaren Stimulators mit Therapieerfolgskontrolle dargestellt. Dieser besteht aus einer Batterie 8a1 und einer Implantats-Elektronik 8a2, die die Komponenten des Blockschaltbildes aus Figur 9a beinhaltet, beides in einem hermetischen Gehäuse untergebracht. An der Unterseite des Gehäuses ist eine Lichtquelle 8a3 angebracht, die ebenfalls hermetisch versiegelt ist, jedoch so, dass das Frequenzspektrum dieser Lichtquelle die hermetische Versiegelung durchdringen und das Zielgewebe -hier Myokard- 8a5 erregen kann. Die Fixierung des Implantates erfolgt hier mit einer Helix 8a4 im Myokard 8a5. In diesem Ausführungsbeispiel umfasst die Implantats-Elektronik 8a2 zusätzlich ein 3D-Beschleunigungssensor 8a6, der zur Therapieerfolgskontrolle derart eingesetzt wird, dass nach jeder optischen Stimulation ausgewertet wird, ob eine Beschleunigung des am Myokard fixierten Implantates festgestellt wird. In diesem Falle gilt die Stimulation als effektiv, da eine Kontraktion des Herzgewebes zur Beschleunigung führt. Bleibt die Beschleunigung aus, gilt die Stimulation als ineffektiv und wird z.B. mit höherer Amplitude wiederholt oder alternativ eine ineffektive Stimulation an ein Fernüberwachungssystem signalisiert.

In der Figur 9a ist das Blockschaltbild des implantierbaren Stimulators 9a0 dargestellt. Dieses Umfasst eine Energiequelle 9a1, ein Sensorinterface 9a2 zur Wahrnehmung eines eine Herzaktion repräsentierenden Merkmals, verbunden mit einer Wahrnehmungseinheit 9a3, die wiederum die Wahrnehmung von Herzaktionen an die angeschlossene Steuereinheit 9a4 signalisiert. Diese Steuereinheit 9a4 ist mit einem Therapiegenerator 9a5 verbunden, welcher bei Erhalt eines Triggersignals von der Steuereinheit 9a4 eine an den Therapiegenerator angeschlossene LED 9a6 anregt und damit ein das Myokardgewebe stimulierendes Lichtsignal aussendet. Der Therapiegenerator 9a5 kann dabei das Lichtsignal in seiner Intensität, Dauer, Signalform und Farbe variieren.

Die Wahrnehmungseinheit 9a3 ist ferner mit einem 3D-Beschleunigungssensor 9a7 verbunden, der zur Therapieerfolgskontrolle derart eingesetzt wird, dass nach jeder optischen Stimulation ausgewertet wird, ob eine Beschleunigung des am Myokard fixierten Implantates festgestellt wird. In diesem Falle gilt die Stimulation als effektiv, da eine Kontraktion des Herzgewebes zur Beschleunigung führt. Bleibt die Beschleunigung aus, gilt die Stimulation als ineffektiv und wird z.B. mit höherer Intensität, Dauer, alternativer Signalform oder anderer Farbe wiederholt.

Figur 10a zeigt beispielhaft zweidimensionale Charakteristiken elektromagnetischer Strahlers mit räumlich selektiver Wirkung.

In der Figur 8b ist eine mögliche Ausführungsform eines implantierbaren Defibrillators dargestellt. Dieser besteht aus einer Hochleistungs-LED 8b1 und einer verkapselten Implantatselektronik nebst Energiequelle 8b2, die die Komponenten des Blockschaltbildes aus Figur 3 beinhaltet, beides in einem hermetischen Gehäuse untergebracht. An der Unterseite des Gehäuses ist eine weitere lokale Lichtquelle 8b3 angebracht, die derart angebracht und dimensioniert ist, dass diese nur einen lokale Depolarisation im vorbehandelten Myokard 8b5 auszulösen vermag. Die Fixierung des Implantates erfolgt hier mit einer Helix 8b4 im Myokard 8b5. Die Hochleistungs-LED 8b1 ist derart dimensioniert, das diese zum Zwecke der Defibrillation nahezu das ganze Herz "durchscheinen" kann, so dass eine gleichzeitige Depolarisation aller erregbaren Myokardzellen im Augenblick der Defibrillation möglich ist.

In der Figur 9b ist der implantierbare Stimulator aus Figur 8b dargestellt, jedoch mit einer alternativen Fixiervorrichtung 9b4, 9b4` in Form von seitlich angebrachten Widerhaken aus Nitinol. An der Unterseite des Gehäuses ist eine Lichtquelle 9b3 angebracht, die hermetisch versiegelt ist, derart, dass das Frequenzspektrum dieser Lichtquelle die hermetische Versiegelung durchdringen und das Zielgewebe -hier Myokard- 9b5 erregen kann.

In der Figur 10b ist das Blockschaltbild des implantierbaren Stimulators 10b0 gemäß Figur 8b dargestellt. Dieses Umfasst eine Energiequelle 10b1, ein Sensorinterface 10b2 zur Wahrnehmung eines eine Herzaktion repräsentierenden Merkmals, verbunden mit einer Wahrnehmungseinheit 10b3, die wiederum die Wahrnehmung von Herzaktionen an die angeschlossene Steuereinheit 10b4 signalisiert. Diese Steuereinheit 10b4 ist mit einem Therapiegenerator 10b5 verbunden, welcher bei Erhalt eines Triggersignals von der Steuereinheit 10b4 eine an den Therapiegenerator angeschlossene LED 10b6 anregt und damit ein das Myokardgewebe stimulierendes Lichtsignal aussendet. Der Therapiegenerator 10b5 kann dabei das Lichtsignal in seiner Intensität, Dauer, Signalform und Farbe variieren.

In der Figur 11 wird eine typische Realisierung dargestellt. Das Implantat 110 ist mit einer Fixiereinrichtung 113 an Organgewebe befestigt und verfügt über einen elektromagnetischen Strahler 111 der mittels der Maske 112 maskiert ist, um nur innerhalb der Wirkkegel 114 und 1141 elektromagnetische Energie auf das zu therapierende Gewebe 115 einstrahlen zu lassen.

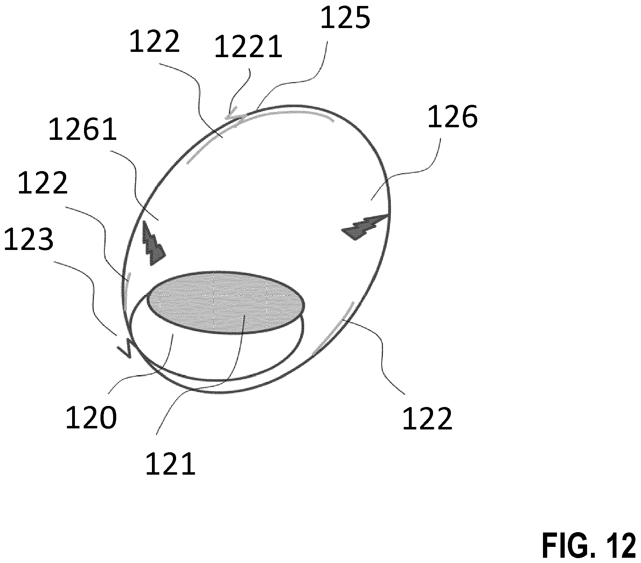

In der Figur 12 ist die Realisierung offenbart, bei der die Maske 122 eine zweite Einheit darstellt und unabhängig vom Implantat mit einer Fixiervorrichtung 1221 befestigt ist, um zu verhindern dass elektromagnetische Energie in die abgedeckten Geweberegionen eindringt. Das Implantat 120 ist mit einer Fixiereinrichtung 123 an Organgewebe befestigt und verfügt über einen elektromagnetischen Strahler 121 der mittels der Maske 122 maskiert ist.. Die Therapie wirkt nur in den nichtmaskierten Regionen 126 und 1261 auf das zu therapierende Gewebe 125.

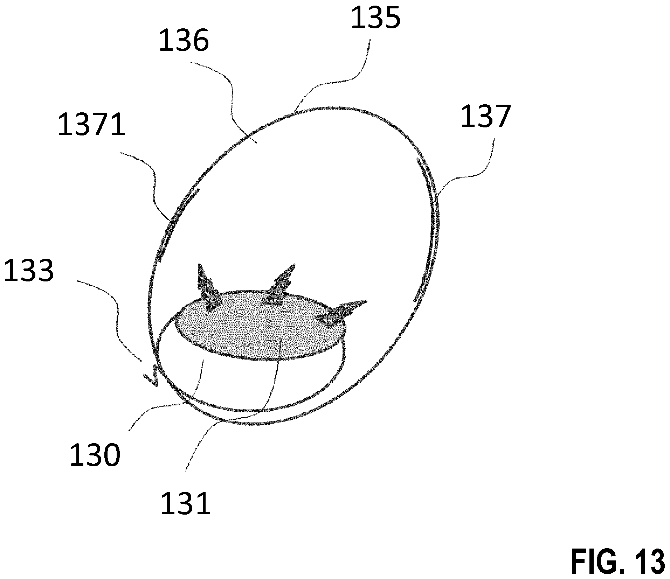

Figur 13 zeigt eine Realisierung mit lokal vorbehandeltem Gewebe. Der Wirkbereich 136 der Strahlung erreicht zwar das gesamte Gewebe 135, wirkt jedoch nur den in den Regionen 137 und 1371. Das Implantat 130 ist mit einer Fixiereinrichtung 133 an Organgewebe befestigt und verfügt über einen elektromagnetischen Strahler 131. Die Therapie wirkt nur in den vorbehandelten Regionen 137 und 1371 auf das zu therapierende Gewebe 135.

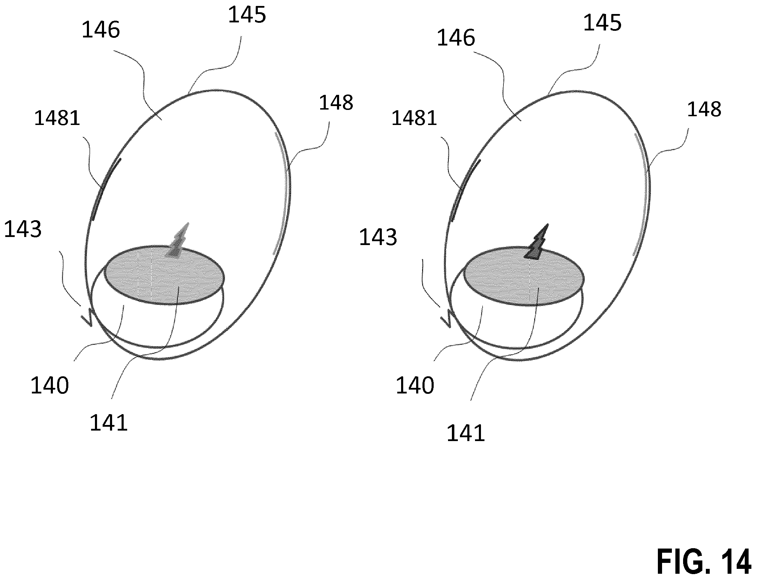

Figur 14 offenbart eine Lösung mit lokal frequenzspezifisch (oder polarisationsspezifisch) reagierendem oder dahingehend vorbehandeltem Gewebe. Je nach Frequenz(band) bzw. Polarisation des Strahlers zeigt nur die eine 148 oder die andere 1481 Region Wirkung. Der Wirkbereich 146 der Strahlung erreicht zwar das gesamte Gewebe 145, wirkt jedoch nur den in den Regionen 148 und 1481. Das Implantat 140 ist mit einer Fixiereinrichtung 143 an Organgewebe befestigt und verfügt über einen elektromagnetischen Strahler 141.

Figur 15 zeigt eine Lösung mit distal zum Implantatgehäuse liegenden Strahlern 151 und 1511 die entweder direkt (nicht gezeigt) oder mittels eines Trägers 154 mit Fixierung 156 an des zu therapierende Gewebe angebracht sind. Dabei übernimmt der Träger auch die Rolle einer Maske und schattet das restliche Organ ab. Die Strahler werden vom Implantat 150 über eine Leitung 1515 versorgt. Alternativ kann das Implantat selbst die Rolle eines Trägers übernehmen. Die Strahler sind dann direkt am Gehäuse befestigt. Das Implantat 150 ist mit einer Fixiereinrichtung 153 an Organgewebe befestigt.

In der Figur 16 ist der Ablaufplan der Methode für das Stimulationssystem gezeigt. Nach Start 160 erfolgt die Vorbehandlung 161 gefolgt von der Implantation und Befestigung des Stimulators 162. Danach wird der Test 163 der Therapiewirksamkeit durchgeführt. Ist dieser nicht erfolgreich wird im Vorgang 164 nachgebessert. Anderen falls ist die Methode beendet 165.

Figur 17 zeigt das Gewebe175 mit den Vorbehandelten Bereichen 177 und 1771. Der Stimulator 170 mit elektromagnetischem Aktuator 171 wird mit der Fixierung 173 am Gewebe befestigt.

Figur 18 zeigt das Gewebe185 mit den durch Vorbehandlung maskierten Bereichen182. Es sind somit nur die Bereiche 186 und 1861 für die vom Aktuator 181 abgegebenen Strahlung stimulierbar. Das Implantat 180 ist mit einer Fixiereinrichtung 183 an Organgewebe befestigt und verfügt über einen elektromagnetischen Strahler 181.

Figur 19 zeigt ein Beispiel mit lokal vorbehandelten Bereichen 198 und 1981 die jedoch für unterschiedlichen Frequenzen der elektromagnetischen Strahlung empfindsam sind.

Die selektive Wirkung der Therapie kommt dadurch zustande, dass der Aktuator 191 elektromagnetische Strahlung einmal auf der einen Frequenz 199 und ein anderes Mal auf einer anderen Frequenz 1991 abgibt. Der Wirkbereich 196 der Strahlung erreicht zwar das gesamte Gewebe 195, wirkt jedoch nur den in den Regionen 198 und 1981. Das Implantat 190 ist mit einer Fixiereinrichtung 193 an Organgewebe befestigt und verfügt über einen elektromagnetischen Strahler 191.

Figur 20 zeigt ein Beispiel für eine selektive Therapie durch Platzierung der Aktuatoren so dass anatomische Beschaffenheiten des Gewebes zur Abschattung genutzt werden. Figur 20 zeigt wie selektive Therapie erreicht wird indem durch Platzierung der Aktuatoren 201 und 2011 (mit dem Stimulator 200 verbunden durch Leitungen 2015 und 2016) anatomische Formen 202 des Gewebes 205 zur Abschattung genutzt werden. Das Implantat 200 ist mit einer Fixiereinrichtung 203 an Organgewebe befestigt.

In der Figur 21 ist das Blockschaltbild eines Stimulators mit optischem Therapieausgang dargestellt. Der Stimulator 210 umfasst eine Energiequelle 211, mindestens einen Energiespeicher 214, eine Vorrichtung zur Ladung 213 des Energiespeichers 214, eine Vorrichtung zur Freigabe der Energie 215. Des Weiteren ist dargestellt ein Aktuator 216, wobei der Aktuator über eine Lichtquelle 218, sowie einen Strombegrenzer 217 (z.B. Widerstand oder Diode) verfügt. Der Stimulator 210 verfügt über eine Steuereinheit 212. Stimulator 210 umfasst eine Wahrnehmungseinheit 2125. Aktuator 216 kann als eine von der übrigen erfindungsgemäßen Vorrichtung trennbare Einheit realisiert sein und über eine Schnittstelle 219 zur Kontaktierung verfügen.

In der Figur 22 ist die Pulsentladung eines ICD dargestellt, die einen optischen Therapieaktuator zur Abgabe einer gepulsten Therapie ansteuert.

Beispielsweise können bereits bekannte elektrische Stimulationsvorrichtungen (z.B. Herzschrittmacher, Neuro-Stimulator) mit einem Aktuator für die Aussendung von elektromagnetischer Strahlung ergänzt werden, um eine Stimulation mittels elektromagnetischer Wellen durchzuführen. Abgesehen vom Aktuator bedarf es dabei für das Stimulationsgerät nur geringfügiger bis keinerlei Modifikationen.

Figur 23 zeigt tabellarisch beispielhafte Kombinationen von Stimulations- und Schockvektoren zwischen rechtem Ventrikel RV, Gehäuse der Stimulationsvorrichtung CAN und rechtem Atrium RA. Betrachtet man exemplarisch die zweite und dritte Zeile der Tabelle, so stellt diese eine Kombination zweier Vektoren dar: ein erster Vektor von Gehäuse CAN zum rechten Ventrikel RV für die Stimulation durch einen Aktuator gemäß der Erfindung, sowie einen zweiten Schockvektor zwischen Gehäuse CAN und rechtem Atrium RA. Bei einem handelsüblichen ICD können diese elektrischen Pole realisiert sein durch eine rechtsventrikuläre Schockwendel, das ICD-Gehäuse und eine supraventrikuläre Schockwendel. Ein Vektor führt jeweils von einem ersten durch das Kreuz gekennzeichneten Pol zu einem zweiten. Die Stimulation kann durch elektromagnetische Strahlung über einen Aktuator gemäß der Erfindung durchgeführt werden, oder durch bekannte elektrische Stimulation, oder durch eine Kombination der beiden.

Figur 24 zeigt schematisch eine Vorrichtung umfassend ein Applikationsmittel zur Abgabe einer Substanz an Gewebe. Das Gerät 241 ist im Köper 240 implantiert. Es verfügt über ein Reservoir 242 für die therapeutische Substanz und Zuleitungen 2435, 2425 und 2445. Das Reservoir wird über einen Port 243 befüllt. Die Pumpe pumpt gesteuert von der Steuereinheit 247 die Substanz zur Gerät-Gewebe-Schnittstelle oder Applikationsmittel 245, die über eine optionale Maske 2455 das Organ 246 mit dieser Substanz behandelt. Eine Kontrolleinheit 249 nimmt u.a. den Reservoirstand wahr und meldet diesen über die Telemetrieeinheit 2495 nach außen. Zum Test der Wirksamkeit gibt von 247 gesteuert die Therapieeinheit 248 ein Testsignal 2485 ab. Die Reaktion wird über eine Wahrnehmungseinheit (hier nicht gezeigt) festgestellt und der Kontrolleinheit gemeldet.

Figur 25 zeigt die erfindungsgemäße implantierbare Vorrichtung 250 die mit Fixiereinrichtung 251 am Gewebe 254 fixiert ist und über einen Sensor 252 zur Wahrnehmung von elektromagnetischer Strahlung 256 verfügt, die als primäre Strahlung von den zu beobachtenden (optional vorbehandelten) erregbaren Zellstrukturen 255 ausgeht, wenn diese Aktionspotentiale ausbilden.

In einer alternativen Realisierung/Anwendungsszenario verfügt die implantierbare Vorrichtung 250 über einen zusätzlichen Aktuator 253 für die Erzeugung von elektromagnetischer Strahlung 257. Diese wird von den zu beobachtenden (optional vorbehandelten) erregbaren Zellstrukturen 255 in Abhängigkeit ihrer Aktionspotentiale moduliert und kehrt als sekundäre Strahlung 256 zum Sensor 252 zurück.

In der Figur 26 wird eine Realisierung mit in der Fixiereinheit eingebauten Aktuatoren und Sensoren für elektromagnetische Strahlung offenbart. Die Fixiereinheit 261 besteht hier aus einem Schaft 2611 mit klappbaren, arretierbaren Armen 2612 die z.B. die Sensoren 262 tragen. Die Aktuatoren 263 sind gegenüberliegend z.B. am Gehäuse 260 fixiert. Bei Implantation werden die Arme in Richtung des Schaftes ausgerichtet, durch die

Organwand 268 gestochen und dann umgeklappt und in der Stellung arretiert, dass sie den Aktuatoren jenseits der Wand passend gegenüberliegen

In der Figur 27 wird eine weitere Realisierung mit in der Fixiereinheit eingebauten Aktuatoren 273 und Sensoren 272 für elektromagnetische Strahlung offenbart. Dabei ist die Fixiereinheit 271 der Aktoren 273 und Sensoren 272 als Zwacke 270 ausgeführt. Eine Zuleitung 271 führt zum Gerät (nicht gezeigt).

Figur 28 zeigt beispielhaft einen bevorzugten Implantationsablauf.

Die dargestellten Schritte sind:
- 280 Start der Implantation
- 281 Vorbehandlung des Zielgewebes
- 282 Feststellung des geeigneten Implantationsortes
- 283 Positionierung der Vorrichtung, solange bis der geeignete Implantationsort erreicht ist
- 2835 Nachstellen der Position
- 284 Fixierung
- 285 Parametereinstellung und Start der Wahrnehmungsmethode (inkl. Aufbereitung und Analyse)
- 286 Testung
- 2865 Nachstellen der Parameter
- 287 Ende der Implantation.

Figur 29 zeigt beispielhaft einen bevorzugten Signalverarbeitungsablauf. Die dargestellten Schritte sind
- 290 Start der Wahrnehmung (Messung)
- 291 Verstärkung,
- 2911 Demodulation,
- 2912 analoge Filterung,
- 292 AD-Wandlung,
- 2921 digitale Filterung,
- 2922 Bestimmung der Signalleistung,
- 2923 Schwellwertbestimmung,
- 293 Segmentierung,
- 2931 Ereigniserkennung,
- 2932 Bestimmung von Periodizität,
- 294 Klassifikation von Rhythmen
- 295 Ende der Wahrnehmung (Messung)

Die Erfindung eliminiert ganz oder teilweise die nachteiligen Wirkungen galvanisch eingekoppelter elektrischer Therapieströme für die Therapie von Herzgewebe, neuronalem Gewebe oder Muskelgewebe.

Dieser selektive Therapieansatz eröffnet neue Möglichkeiten für die multifokale Therapie ohne für jeden Stimulationsort eine gesonderte Sonde implantieren zu müssen. Durch die großflächig mögliche multifokale Therapie können Erregungsmuster angeregt werden, die die natürlichen spatio-temporalen Verhältnisse viel besser als bisher abbilden.

Die Anforderungen an den Energiebedarf von derartigen Implantaten kann erheblich gesenkt werden. Es werden ferner völlig neue Bauformen derartiger Implantate möglich.

Im Kontext der Erfindung werden folgende Begriffe als Synonym für die erfindungsgemäße implantierbare Vorrichtung zur Wahrnehmung von elektromagnetischen Wellen, die von genetisch manipuliertem Gewebe ausgesendet werden, und/oder zur Stimulation von genetisch manipuliertem Gewebe mittels elektromagnetischer Wellen verwendet: Stimulator, Stimulationsgerät, Stimulationsvorrichtung, Vorrichtung/Gerät zur Stimulation, Stimulationssystem (Vorrichtung ist zumindest Teil eines solchen beschriebenen Stimulationssystems).

Im Kontext der Erfindung:
wird unter "Wellenzug" eine durchgehende elektromagnetische Welle verstanden;
- werden die Begriffe "Elektromagnetische Strahlung", "Elektromagnetische Welle" als Synonym verwendet
- ATP hat die Bedeutung "Anti tachycardia pacing", IPG hat die Bedeutung "Implantable Pulse Generator", ICD hat die Bedeutung "Implantable Cardioverter-Defibrillator"

## Patentansprüche

1. Vorrichtung (250), mindestens umfassend:
- eine Energiequelle,
- eine Elektronikeinheit,
- eine Aufnahmeeinheit, mit der Elektronikeinheit gekoppelt und dazu ausgelegt, elektromagnetische Wellen im Frequenzbereich 10¹³-10²⁰ Hz zu messen, wobei die Vorrichtung zur Implantation im menschlichen oder tierischen Körper ausgelegt ist,
wobei die Vorrichtung dazu ausgelegt ist, zu detektieren, dass die elektromagnetischen Wellen von genetisch manipuliertem Gewebe abgestrahlt wurden, und
wobei die Vorrichtung einen Aktuator (253) aufweist, der mit der Elektronikeinheit und/oder der Energiequelle gekoppelt ist und der Aktuator dazu ausgelegt ist, elektromagnetische Wellen (257) im Frequenzbereich 10¹³-10²⁰ Hz auszusenden
**dadurch gekennzeichnet, dass**
die Elektronikeinheit dazu ausgelegt ist, zu erkennen, dass durch die Aufnahmeeinheit gemessene elektromagnetische Wellen (256) vom Aktuator ausgesendet
wurden,
wobei es sich bei der durch die Aufnahmeeinheit gemessenen elektromagnetischen Welle um die durch den Aktuator ausgesendete elektromagnetische Welle in veränderter Form handelt, wobei die Veränderung beruht auf mindestens einem der Effekte:
- Reflexion,
- Absorption,
- Transmission, und/oder
- Polarisation.

2. Vorrichtung nach Anspruch 1, wobei es sich bei der durch die Aufnahmeeinheit gemessenen elektromagnetischen Welle um die durch den Aktuator ausgesendete elektromagnetische Welle in veränderter Form handelt, wobei die Veränderung auf dem Effekt der Fluoreszenz beruht.

3. Vorrichtung nach mindestens einem der Ansprüche 1 oder 2, wobei die Elektronikeinheit dazu ausgelegt ist, in der von der Aufnahmeeinheit gemessenen elektromagnetischen Welle mindestens einen der folgenden Parameter, eine Kombination aus diesen oder eine abgeleitete Größe aus diesen zu erkennen:
- Amplitude,
- Frequenz oder Frequenzspektrum, und/oder
- Polarisationsrichtung,
- Phase,
wobei mindestens eines der folgenden Verfahren angewendet wird:
- Modulationsverfahren
- Pulsweitenmodulation,
- Erkennung der Veränderung durch Anwendung von Filtern.

4. Vorrichtung nach mindestens einem der vorigen Ansprüche, wobei die Elektronikeinheit oder Aufnahmeeinheit die gemessene elektromagnetische Welle in ein elektronisches Signal umwandelt und vorverarbeitet mittels mindestens eines der folgenden Verfahren:
- Verstärkung,
- Demodulation,
- Filterung,
- AD-Wandlung,
- Gleichrichtung,
- Bestimmung der Signalleistung,
- Schwellwertbestimmung,
- Transformation in den Frequenzbereich
- Bestimmung von Signalqualität, und/oder
- Bestimmung signalmorphologischer Parameter.

5. Vorrichtung nach mindestens einem der vorigen Ansprüche, wobei die Elektronikeinheit oder Aufnahmeeinheit die gemessene elektromagnetische Welle in ein elektronisches Signal umwandelt und analysiert mittels mindestens eines der folgenden Verfahren:
- Segmentierung,
- Ereigniserkennung,
- Bestimmung von Periodizität,
- Bestimmung der Phasenlage,
- Bestimmung von Stabilität,
- Klassifikation von Rhythmen, und/oder
- Klassifikation von signalmorphologischen Parametern.

6. Vorrichtung nach mindestens einem der vorigen Ansprüche, wobei die Aufnahmeeinheit mindestens einen der folgenden Sensoren aufweist:
- Photodiode,
- Phototransistor,
- CCD Element, und/oder
- analoger oder digitaler Bildsensor.

7. Vorrichtung nach mindestens einem der vorigen Ansprüche, wobei die Vorrichtung mindestens einen ersten und einen zweiten Aktuator umfasst, die elektromagnetische Wellen in unterschiedlichen Frequenzen aussenden.

8. Vorrichtung nach mindestens einem der vorigen Ansprüche 2 bis 9, wobei der mindestens erste Aktuator mit einem Gehäuse der Vorrichtung gekoppelt oder entfernt vom Gehäuse angeordnet ist, und der Aktuator mit der Aufnahmeeinheit verbunden ist.

9. Vorrichtung nach mindestens einem der vorigen Ansprüche, wobei die Vorrichtung eine Fixiervorrichtung aufweist, die dazu ausgelegt ist, die Vorrichtung in menschlichem oder tierischen Gewebe zu fixieren, und die dazu ausgelegt ist, die elektromagnetische Welle durch das Gewebe zu leiten.

10. Vorrichtung nach mindestens einem der vorigen Ansprüche, wobei die Vorrichtung über ein Elektrostimulationsmittel verfügt.

11. Vorrichtung nach mindestens einem der vorigen Ansprüche, wobei die Vorrichtung zum Aussenden elektromagnetischer Wellen an Herzgewebe oder an Nervengewebe im Rückenmark oder Muskelgewebe ausgelegt ist.

12. Vorrichtung nach mindestens einem der vorigen Ansprüche, wobei der Aktuator dazu ausgelegt ist, elektromagnetische Wellen zur Stimulation von genetisch manipuliertem Gewebe auszusenden, wobei die Stimulation im Frequenzbereich 10¹³ bis 10²⁰ Hz liegt.

## Claims

1. Device (250), comprising at least:
- an energy source,
- an electronics unit,
- a pickup unit, coupled to the electronics unit and configured to measure electromagnetic waves in a frequency range 10¹³-10²⁰ Hz,
wherein the device is configured for implantation in the human or animal body,
wherein the device is configured to detect that the electromagnetic waves have been radiated from genetically manipulated tissue, and
wherein the device comprises an actuator (253) coupled to the electronics unit and/or the energy source, the actuator being configured to emit electromagnetic waves (257) in a frequency range 10¹³-10²⁰ Hz
**characterized in that**
the electronics unit is configured to detect that the electromagnetic waves (256) measured by the pickup unit have been emitted by the actuator,
wherein the electromagnetic wave measured by the pickup unit is the electromagnetic wave emitted by the actuator in a changed form, the change being based on at least one of the effects:
- reflection,
- absorption,
- transmission, and/or
- polarization.

2. Device according claim 1, wherein the electromagnetic wave measured by the pickup unit is the electromagnetic wave emitted by the actuator in a changed form, the change being due to the effect of fluorescence.

3. Device according to at least one of claims 1 or 2, wherein the electronics unit is configured to detect in the electromagnetic wave measured by the pickup unit at least one of the following parameters, a combination of them, or a variable derived from them:
- amplitude,
- frequency or frequency spectrum, and/or
- polarization direction,
- phase,
wherein at least one of the following methods being used:
- modulation method
- pulse-width modulation,
- detection of a change by applying filters.

4. Device according to at least one of the preceding claims, wherein the electronics unit or pickup unit converts the measured electromagnetic wave into an electronic signal and preprocesses it by means of at least one of the following processes:
- amplification,
- demodulation,
- filtering,
- AD conversion,
- rectification,
- determination of the signal strength,
- threshold determination,
- transformation into the frequency range
- determination of signal quality, and/or
- determination of signal morphological parameters.

5. Device according to at least one of the preceding claims, wherein the electronics unit or pickup unit converts the measured electromagnetic wave into an electronic signal and analyzes it by means of at least one of the following processes:
- segmentation,
- event detection,
- determination of periodicity,
- determination of the phase position,
- determination of stability,
- classification of rhythms, and/or
- classification of signal morphological parameters.

6. Device according to at least one of the preceding claims, wherein the pickup unit comprises at least one of the following sensors:
- photodiode,
- phototransistor,
- charge-coupled device (CCD) element, and/or
- analog or digital image sensor.

7. Device according to at least one of the preceding claims, wherein the device comprises at least a first and a second actuator emitting electromagnetic waves in different frequencies.

8. Device according to at least one of the preceding claims 2 to 9, wherein the at least first actuator is coupled to or remote from a housing of the device, and the actuator is connected to the pickup unit.

9. Device according to at least one of the preceding claims, wherein the device comprises a fixation device configured to fix the device in human or animal tissue and configured to guide the electromagnetic wave through the tissue.

10. Device according to at least one of the preceding claims, wherein the device has an electrical stimulation means.

11. Device according to at least one of the preceding claims, wherein the device is configured to emit electromagnetic waves to cardiac tissue or to nerve tissue in a spinal cord or muscle tissue.

12. Device according to at least one of the preceding claims, wherein the actuator is configured to emit electromagnetic waves for stimulation of genetically manipulated tissue, wherein a stimulation is in a frequency range from 10¹³ to 10²⁰ Hz.

## Revendications

1. Dispositif (250) comprenant au moins
- une source d'énergie,
- une unité électronique,
- une unité d'enregistrement, couplée à l'unité électronique et conçue pour mesurer les ondes électromagnétiques dans la gamme de fréquences 10¹³-10²⁰ Hz,
le dispositif étant conçu pour être implanté dans le corps humain ou animal,
dans lequel le dispositif est adapté pour détecter que les ondes électromagnétiques ont été émises par un tissu génétiquement manipulé, et
dans lequel le dispositif comprend un actionneur (253) couplé à l'unité électronique et/ou à la source d'énergie, et l'actionneur est adapté pour émettre des ondes électromagnétiques dans (257) la plage de fréquences 10¹³-10²⁰ Hz
**caractérisé en ce que**
l'unité électronique est adaptée pour détecter que des ondes électromagnétiques (256) mesurées par l'unité d'enregistrement ont été émises par l'actionneur,
dans lequel l'onde électromagnétique mesurée par l'unité d'enregistrement est l'onde électromagnétique sous une forme modifiée émise par l'actionneur, la modification étant due à au moins un des effets :
- réflexion,
- l'absorption,
- transmission, et/ou
- polarisation.

2. Dispositif selon la revendication 1, dans lequel l'onde électromagnétique mesurée par l'unité d'enregistrement est l'onde électromagnétique sous une forme modifiée émise par l'actionneur, la modification étant due à l'effet de la fluorescence.

3. Dispositif selon au moins l'une des revendications 1 ou 2, dans lequel l'unité électronique est adaptée pour détecter dans l'onde électromagnétique mesurée par l'unité d'enregistrement au moins l'un des paramètres suivants, une combinaison de ceux-ci ou une grandeur dérivée de ceux-ci:
- amplitude,
- fréquence ou spectre de fréquence, et/ou
- direction de polarisation,
- phase,
dans lequel au moins l'un des procédés suivants est utilisé :
- procédure de modulation
- modulation de largeur d'impulsion,
- détection du changement par l'application de filtres.

4. Dispositif selon au moins l'une des revendications précédentes, dans lequel l'unité électronique ou l'unité d'enregistrement convertit et prétraite l'onde électromagnétique mesurée en un signal électronique au moyen d'au moins l'un des procédés suivants:
- renforcement,
- démodulation,
- filtrage,
- conversion AD,
- redressement,
- détermination de la puissance du signal,
- détermination de la valeur seuil,
- transformation dans le domaine fréquentiel
- détermination de la qualité du signal, et/ou
- détermination des paramètres morphologiques du signal.

5. Dispositif selon au moins l'une des revendications précédentes, dans lequel l'unité électronique ou l'unité d'enregistrement convertit et analyse l'onde électromagnétique mesurée en un signal électronique au moyen d'au moins l'un des procédés suivants:
- segmentation,
- détection d'événements,
- détermination de la périodicité,
- détermination de la position de phase,
- détermination de la stabilité,
- classification des rythmes, et/ou
- classification des paramètres morphologiques du signal.

6. Dispositif selon au moins l'une des revendications précédentes, dans lequel l'unité de réception comprend au moins l'un des capteurs suivants :
- photodiode,
- phototransistor,
- élément CCD, et/ou
- capteur d'image analogique ou numérique.

7. Dispositif selon au moins l'une des revendications précédentes, dans lequel le dispositif comprend au moins un premier et un deuxième actionneur émettant des ondes électromagnétiques à des fréquences différentes.

8. Dispositif selon au moins l'une des revendications 2 à 9 précédentes, dans lequel l'au moins premier actionneur est couplé à un boîtier du dispositif ou est situé à distance du boîtier, et l'actionneur est relié à l'unité de réception.

9. Dispositif selon au moins l'une des revendications précédentes, dans lequel le dispositif comprend un dispositif de fixation qui est adapté pour fixer le dispositif dans un tissu humain ou animal et qui est adapté pour guider l'onde électromagnétique à travers le tissu.

10. Dispositif selon au moins l'une des revendications précédentes, dans lequel le dispositif dispose d'un moyen d'électrostimulation.

11. Dispositif selon au moins l'une des revendications précédentes, dans lequel le dispositif est conçu pour émettre des ondes électromagnétiques vers le tissu cardiaque ou vers le tissu nerveux dans la moelle épinière ou le tissu musculaire.

12. Dispositif selon au moins l'une des revendications précédentes, dans lequel l'actionneur est adapté pour émettre des ondes électromagnétiques pour stimuler un tissu génétiquement manipulé, la stimulation étant dans la plage de fréquences de 10¹³ à 10²⁰ Hz.
